# EUROPEAN PATENT APPLICATION

(11) **EP 4 578 942 A1**
(43) Date of publication of application: **02.07.2025**
(21) Application number: 24222992.0
(22) Date of filing: 23.12.2024
(51) Int. Cl.: C12N 5/0775, A61L 27/38

(54) **METHOD FOR OBTAINING STEM CELLS FROM DENTAL PULP**

(30) Priority: 28.12.2023 PL 44731523
(71) Applicant: Uniwersyteckie Centrum Stomatologiczne, 50-425 Wroclaw (PL); Uniwersytet Medyczny Im. Piastów Slaskich We Wroclawiu, 50-367 Wroclaw (PL)
(72) Inventor: Dominiak, Marzena, Tyniec Maly (PL); Gedrange, Tomasz, Wroclaw (PL); Gebarowski, Tomasz, Malin (PL); Hadzik, Jakub, Wroclaw (PL); Kuzniarski, Amadeusz, Olawa (PL); Barg, Ewa, Wroclaw (PL); Cwynar-Zajac, Lucja, Wroclaw (PL); Gebczak, Katarzyna, Wroclaw (PL); Moreira, Helena, Wroclaw (PL); Pitulaj, Artur, Wroclaw (PL)
(74) Representative: Pawlowska-Bajerska, Justyna

(57) **Abstract**

The invention refers to efficient method of obtaining stem cells from dental pulp, including the stage of isolating cells from the pulp and multiplying them, allowing the production of material that can be used in the production of tissue engineering medicinal product. The invention relates to a method of obtaining, including the isolation of stem cells from dental pulp, proliferating them for the production of tissue engineering product. The purpose of the method of producing dental pulp stem cells is to use them as an active substance in advanced therapy medicinal products, in particular in tissue engineering products intended for the treatment of bone tissue defects - cavities.

## Description

The subject of invention refers to efficient, productive *in vitro* method for obtaining stem cells from cells derived from dental pulp tissue pulp for medical - therapeutical use, comprising the step of isolating cells from pulp and proliferating them, enabling the production of material - product that is for use in the production of medical products, therapeutical, pharmaceutical products of tissue engineering. The invention refers to method of obtaining with the isolation of stem cells from dental pulp - cells derived from dental pulp, proliferating of cells for the production of a tissue engineering product - material. The purpose of the method of obtaining - producing stem cells of dental pulp is to use the obtained product - the cells as an active substance, biological active product in medical products, therapeutical products in advanced therapy, in particular in tissue engineering products intended for use in therapy, treatment of bone tissue declines, cavities.

The processes of bone and soft tissue regeneration are subject to significant limitations. In order to achieve the necessary clinical results in the case of extensive defects, declines, it is requirement to use stem cells to rebuild bone mass in the area of the defects, declines resulting from surgical intervention. The obtains, production of the stem cells in adults is currently very limited.

Stem cell isolation was described in the 1990s. However, the efficiency of these methods is very limited, and obtaining healthy stem cells from teeth was quite poor. In order to achieve of regenerate bone, a large number of them is required. Multiplying - proliferating the obtained stem cells alone have not gave the appropriate, expected results. For this reason, the evidence described in the state of the art, literature was enabling, only suitable for scientific research and not susceptible of industrial application, for the development of commercial therapy.

Known methods - protocols do not lead to the production of a product in accordance with the requirements of Good Manufacturing Practice (GMP). Therefore, cells obtained as a result of known methods cannot be used in experiments on patients, clinical trials or products for use in medicine.

The aim, purpose of the invention was to provide productive - effective method of isolating stem cells based on optimization in order to increase the number of stem cells from tooth - dental pulp. To achieve the goal the scientific team with extensive experience in the field of oral medicine - stomatology, together with a team of scientists from biological fields, based on medical, practical and scientific knowledge and research - laboratory experience, provided new method, protocol from the isolation of cells from dental pulp, storage them, and proliferation of the stem cells, enabling, allowing the obtaining, production of the stem cells that can be used in stomatologic therapy, dental therapy, including stomatologic, dental surgery, treatment of bone defects - declines, cavities, including obtains - receipt of implants.

The aim of the invention is to produce, obtain tissue engineering product, material for use in tissue regeneration in stomatology, dentistry.

In the first step - stage, pulp is obtained from a tooth, teeth - derived from dental pulp. The substrate in the method - source of dental pulp, according to the invention is the obtained tooth or teeth, especially molars tooth/teeth removed from patients in the extraction process. Stem cells from the dental, tooth pulp can be obtained from baby teeth or adult teeth obtained during standard stomatology, dental procedures, such as tooth extraction, which makes the method easier and more ethical to obtain the substrate. After extraction of the tooth or teeth and therefore removal of the entire tooth, which is not a step of the invention, its decontamination is performed according to the invention using PBS buffer without Ca, Mg ions, with the addition of such an amount of antibiotic, especially gentamicin, in order to conduct the cell culture without infection - preventing cell culture decontamination, preferably a solution of 50 mg / ml and this amount is 0.5 I of PBS in a volume of 20 ml for each tooth, while the decontamination is performed at room temperature. Then, in order to obtain live- active dental pulp - tooth pulp, the step of cutting a tooth *in vitro* under sterile conditions is performed while the cutting is conducted at the level of the dental enamel-cement junction in order to not damage the cavity of dental/tooth pulp during this process - the location of cutting enable to obtain active dental pulp.

If the pulp for culture is to be transported, the obtained pulp is placed in a container/tube in medium for transporting known in state of the art in accordance with the requirements of biotechnology or pharmacy, especially in alpha MEM (minimal essential medium) comprising 20% FBS and with the addition of gentamicin and/or fungizone, stable L-glutamine in a airtight - hermetic container or tube, e.g. 50 ml Falcon type tube, e.g. in the composition of alpha MEM (Sartorius) with the addition of 20% FBS (according to the requirements of the current, in force European Pharmacopoeia, intended for use in biotechnology or pharmacy - Biopharm grade or Pharma grade), and with the addition of gentamicin (50 mg) fungizone, stable L-glutamine. All reagents must be manufactured in accordance with the GMP standard and proper in the field - to be intended for future manufacturing or for use in the manufacturing process. It is not permissible to use materials intended exclusively for research - they do not guarantee the appropriate quality of the final product. The pulp obtained in this manner can be stored at a controlled temperature of from 2 up to 8 °C and delivered to the laboratory within 72 hours - until, not more than 72 hours.

Further steps of the method can be performed in special laboratory, manufactory with perdition, certification for obtaining, producing of products for use in medicine, pharmaceutical product for advanced therapy. Further steps of the process must be carried out in a class A isolator. The surface of container or tube is disinfected by hydrogen peroxide evaporating or using known product for sterilizing, disinfection, known disinfectant, e.g. 70% isopropanol solution.

From this stage, the main steps of obtaining and proliferating stem cells from dental pulp according to the invention starts.

The pulp is placed in a tube with a volume adjusted to the amount of material - obtained pulp at the previous step, e.g. a 5 ml tube and the step of mechanical disintegrating using known, disinfected - sterile mean - scissors is performed. PBS buffer without Ca, Mg ions is added to the disintegrated tissue with the addition of collagenase enzyme in amount adjusted to the volume of the pulp in order to obtain cells from the pulp by enzymatic digestion, e.g. - 4 mg/ml. Digesting step of obtained mixture is performed. As the added collagenase is pharmaceutical material - substrate used in the method and it is prepared by dissolving in PBS and then filtering through filter, e.g. 0.22 µm in size. The suspended pulp is incubated for at least 30 minutes, preferably 1 hour at 37°C in incubator, stirring gently - mixing. Mixing the solution is necessary for achievement of proper digestion, while mixing should be performed during digestion step at a frequency of up to 200 RPM. After the incubation time, complete cell growth medium is added to the obtained suspension in a ratio of 1:1 to the volume of the digestion mixture. Then, the mixture is centrifuged at a temperature from 4 up to 20 °C, preferably at 400 g for 6 minutes. After centrifugation, the supernatant is removed, and the cell pellet - sedimented cells - cell sediment is suspended in a selected complete culture medium for the mesenchymal stem cells culturing with the addition of fetal bovine serum in a selected amount 20% FBS in 1 ml of medium, gentamicin and fungizone in an amount adjusted in order to not lead to infection of the cell culture, in order to conduct cell culture at a sterile condition, e.g. in the composition: MSC medium, e.g. Nutristem - Basal Medium 500 ml + Supplement Mix (Sartorius), fetal bovine serum FBS 20% Biopharm grade or Pharma grade, gentamicin (50 mg/l), fungizone. Before cell seeding, culture vessels are prepared and coated with a known in the art mean - substance, preparation, e.g. MSC Attachment Solution or mean comprising human fibronectin for production of pharmaceuticals, drugs. The cell suspension is seeded to culture vessel, e.g. TPP or another one that meets GMP requirements, coated with MSC Attachment Solution.

Then, the prepared at previous step culture of dental pulp cells prepared is carried out at a temperature of 37°C, in atmosphere comprising 5% CO₂ until the cells reach 60-80% of confluence, changing the heretofore mentioned medium for a maximum of 72 hours - for a duration up to 72 hours. The cell culture step is carried out in incubator suitable for this type of culture, e.g. a class A area, in incubator built into the isolator. In the case when cell culture is carried out from material obtained from several patients, in the culture process, the culture vessels must be additionally placed in known barrier mean, systems, separating chambers such as IsocellBIOBOX or CondoCell.

Additionally, maintaining all quality requirements, at the provided steps according to the invention with regard to sterility and for use in therapeutic purposes is necessary to obtain during the method product for use in medicine, treatment as a result.

The provided process of cell isolation from pulp and process of cell culture is significantly different from others known in the art and enables to obtain product for use in regeneration of tissues in human being. Additionally, it enables to conduct cell culture of material collected from several patients at the same time.

After obtaining of 60-80% cell confluence during the culture, it is necessary to perform the cell passage procedure - detachment. Further step of cell culture is performed in uncoated culture vessels, i.e. subsequent stages are performed in the same culture medium but without coating. Coating at the previous culture step enables to achieve better adhesion to the growth surface of tissue fragments from which the cells from used dental pulp migrate while after the passage step this is no longer necessary.

After that, in order to detach the cells for therapeutic use, reagent for enzymatic detachment of cells without CaCl₂ and MgCl₂ is used, e.g. CTS series (Cell Therapy Systems) CTS^{™} DPBS (1X), without calcium chloride, without magnesium chloride to rinse the growth surface and CTS^{™} TrypLE^{™} Select Enzyme for enzymatic detachment of cells. The process begins with at least one rinse of the layer of cultured cells obtained in the previous step with PBS solution, and then incubation in reagent containing enzyme for detachment of cells from the substrate, the process is performed until detachment but not destruction of stem cells, e.g. for 5 minutes at 37°C, in TrypLE. Alternative reagents can also be used for the detachment process - biotechnological trypsin and PBS, if the quality requirements for obtaining cells for therapeutic use, especially GMP, are met. After the detachment time - after obtain of the detached cells, the cells are visible under the microscope as non-adherent - they stop adhering to the substrate of the culture vessel. Equal volume of the culture medium for mesenchymal stem cells mentioned above at the cell culture step - the same as mentioned at the previous stage - is then added to the suspension in order to neutralize the enzyme for detachment. The cell suspension is then transferred to next, subsequent centrifuge tube to obtain centrifuged cells, e.g. using conical tube and centrifuged to obtain cell sediment - pellet, e.g. at 400g for 6 minutes. Then the cells are suspended in the same medium for isolation and culture of mesenchymal cells mentioned above, at the first step of culture, and their number is counted. Cell counting is performed in a known manner, e.g. for counting, a sample is taken for counting in a NucleoCounter^{®} model NC-200 or higher or another cell counter compliant with the GMP standard. The use of an appropriate cell counter, e.g. the one mentioned above, is essential to perform correct measurement in accordance with GMP requirements.

The obtained cell suspension is placed again in culture vessels with a surface area at least 3x larger than the previously used vessel, e.g. from 25 cm² bottle cells are transferred to 75 cm² or other ones that meet the standards for conducting cell cultures for therapeutic purposes.

Depending on the number of cells obtained, the next step is carried out - the step of cell culture in medium for isolation and culturing of hMSC mesenchymal cells, for conducting of culture of mesenchymal stem cells with addition of fetal bovine serum in an amount of at least 20% FBS in 1 ml of medium, gentamicin and fungizone in an amount that does not lead to infection of the culture (enable to perform culturing in sterile condition), e.g. in the composition: MSC medium, e.g. Nutristem - Basal Medium 500 ml + Supplement Mix (Sartorius), fetal bovine serum FBS 20% Biopharm grade or Pharma grade, gentamicin (50mg/I), fungizone. This stage, however, is carried out without coating like the first step of cell culture.

This step is continued until 60-80% of cell confluence is achieved. Cell culture is continued until 60-80% confluence is achieved.

The quality assessment of the obtained mesenchymal stem cells is performed using a known laboratory technique, preferably flow cytometry by assessing the cell phenotype, e.g. using the DURAClone SC Mesenchymal Stem Cell kit using by flow cytometry in accordance with the guidelines - instruction included in the kit information. The use of antibodies to assess the markers specifying stem cell phenotype, that enable to mark the specific molecules on their surface allows for a repeatable assessment of the number of obtained stem cells.

The obtained cells can be used to produce a tissue engineering product, can be banked or evaluated - measured, marked. In the case when obtained cells are to be feezed, the obtained cell shall be suspended in medium that meets the requirements of the standards for conducting/performing cell culture for therapeutic purposes, especially GMP standards in connection with the possibility of the cells reuse in the production process. For freezing, cell pellet - cell sediment is suspended in a known preparation for freezing stem cells, e.g. NutriFreez. Cell suspension of a given concentration, depending on the number of stem cells required for using in particular type of treatment, e.g. at a concentration of 10⁶/ml cells, is transferred to appropriate freezing tubes, i.e. cryovials, which are placed in a CoolBOX container enabling to reach controlled freezing conditions and tubes are cooled to -80°C with a freezing rate of -1°C/min. Then the cryovials are placed in dewars with liquid nitrogen, where they can be stored for a long time at -196°C.

In comparison to known methods of isolating and culturing stem cells from dental pulp for therapeutic purposes, the presented invention enables to proper obtain of the appropriate - selected group of mesenchymal stem cells for therapeutic purposes. In particular, the following, most significant parameters - condition of isolation method itself and cell culture have been established.

The proper obtained pulp *in vitro* according to method, is subjected to enzymatic digestion using PBS without Ca, Mg ions with the addition of collagenase enzyme in an amount adjusted to the pulp volume in order to obtain cells from the particular amount of pulp by enzymatic digestion with 4 mg/ml. Digestion process is carried out by incubating pulp in said mixture for at least 30 minutes at 37°C in an incubator and with gentle stirring - mixing during this.

Additionally, the composition of the cell culture medium was selected according to the invention - i.e. cell growth medium, which is used in a 1:1 ratio to the volume of the mixture after digestion - it is a culture medium for the culture of mesenchymal stem cells with the addition of fetal bovine serum in a selected amount, i.e. at least 20% FBS in 1 ml of medium, addition of gentamicin and fungizone in an amount that does not lead to infection of the culture - enable to conduct cell culture in sterile condition. Coating the culture medium at this step of the cell culture enables, allows for better adhesion to the growth surface of tissue fragments from which the dental pulp cells migrate. Step I of the culture is carried out using such coating using known component for coating od surface of the culture vessels. Next, subsequent stages of the culture step from passage are carried out without coating.

It was developed, experimentally checked and provided according to the invention that the cell culture - the step of growing of cells are carried out until 60-80% of cell confluence is obtained, and then the passage procedure is performed and further cell culture is performed in culture vessels that do not require prior coating - without of the coting of surface of vessels.

The most favorable parameters - time and condition are indicated above, preferably the passage step is carried out for 5 minutes at 37°C.

The method enables to obtain the dedicated, proper - eligible cells from tooth - dental pulp, which can be used for therapeutic, treatment purposes in dental surgery, stomatology surgery. The method enable to obtain such amount of mesenchymal stem cells that is sufficient to carry out therapy, which is required, desirable, seeking.

In addition, other additional steps have been provided according to the invention that can be used - the following modifications:
1. The composition of transport medium was developed, ensuring an extended safe transport time of isolated pulp up to 72h.
2. Proper medium for cell isolation and culture was selected, thanks to which a homogeneous culture is obtained, and therefore cell sorting is not necessary. Over 80% of the cells meet the criteria for MSC cells. The results obtained thanks to the provided method are significantly better than other isolation methods.
3. Modified growth surface is used at the isolation step, which improves cell adhesion to the growth surface. Coating the culture medium with medium of the selected composition is at the isolation step.

Stem cells from dental pulp have been currently widely studied and can be used in stomatology, dentistry and dental, stomatologic surgery. Their unique properties, such as the ability to self-renew and differentiate into different cell types, make them promising therapeutic tools in the regeneration of dental tissues. Stem cells from dental pulp also have the potential to regenerate bone in the oral cavity. They can be used to treat bone defects, bone deficit, cavities resulting from periodontal disease or after trauma. Finally, the use of stem cells from dental pulp can help to avoid the controversial use of fetal stem cells - preventing from such use. The invention enables to use of obtained stem cells from dental pulp in stomatology, dentistry and dental surgery and this make the invention promising tools. Their ability to regenerate tissue and unique properties make them an attractive therapeutic tool in the treatment of oral tissue diseases.

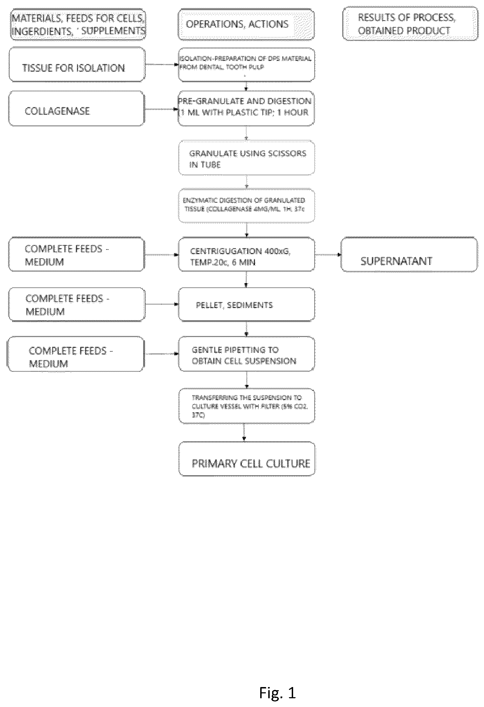

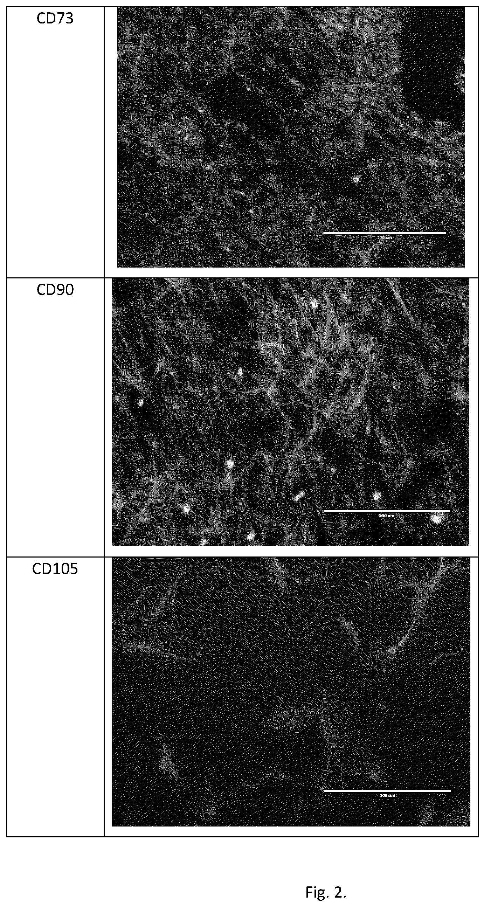

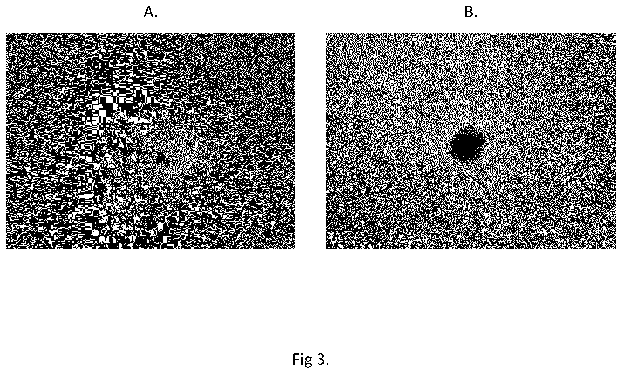

The method according to the invention and concerning the storage of non-embryonic stem cells is presented in examples - embodiments, in the block diagram presenting steps of the process of isolation of dental pulp cells and in the drawing where fig. 1 shows a block diagram of the process of isolation of dental pulp cells - stages, fig. 2 - shows a visualization of the expression of selected surface antigens in cells isolated from dental pulp and fig. 3 -a microscopic photo from a primary culture of cells isolated from dental pulp. A. 72h from isolation, 7 days from isolation. Detailed description of the drawing: Fig. 1. Block diagram of the process of isolation of cells from dental pulp. Fig. 2 microscopic photo using the cell marker CD73, CD90, CD105. Fig. 2. Expression of selected surface antigens in cells isolated from dental pulp. Fig. 3. Primary culture of cells isolated from dental pulp. A. 72h from isolation, B. 7 days from isolation.

### Example

### Cells obtained from the dental pulp - pulp of a tooth obtained during extraction.

A tooth, e.g. an adult molar after extraction, was placed in a tube with 20 ml of PBS/gentamicin for decontamination. The tooth was then cut and the pulp obtained was transported in transport medium in the composition of alpha MEM (minimal essential medium) containing 20% FBS and with the addition of gentamicin and fungizone, stable L-glutamine. The used in the method teeth can't have advanced caries - advanced decays, must be living, alive teeth, and their structure must be preserved in its entirety during the extraction procedure. The biological material in the tube and the medium for transport was placed in a tissue transport refrigerator at 4°C, in order to maintain, keep a transport temperature of 2-8°C. The material was transported to the Cell and Tissue Bank together with the necessary documentation. After tooth extraction, its decontamination was performed using PBS buffer without Ca, Mg ions, with the addition of such an amount of antibiotic, especially gentamicin, that it is possible to conduct culture without infection, in the example 50 mg/ml solution and this amount was 0.5 I of PBS in a volume of 20 ml per the extracted tooth, at room temperature. Then, in order to obtain the living dental pulp of the tooth, it was cut in sterile conditions at the height of the enamel-cement junction - cementoenamel junction, so as not to violate, invade - destroy the dental pulp chamber of the tooth during this process.

After registration at the Tissue and Cell Bank, the material was transferred to the ATMP centre - laboratory plant/manufactory. Cells from dental pulp were isolated under sterile conditions in class A, in an isolator in a class D environment. The entire pulp obtained from the tooth was placed in a test tube and mechanically granulated, crumbled into smaller pieces of tissue using sterile scissors. The crumbled tissue was placed in a test tube and digested with 2 ml of a 4 mg/ml collagenase enzyme solution (pharma grade) for 1 hour at 37°C, shaking. After the incubation time, the suspension was mixed, centrifuged at room temperature at 400 g for 6 minutes. The supernatant was removed and the cell pellet - sediment was suspended in 5 ml of culture medium. The cell suspension obtained in the digestion process was seeded into, to a culture vessel previously covered with MSC Attatment solution - the cells was spread to the vessel, as described earlier, which increases cell adhesion - stage I of the cell culture. After 72 hours. the culture medium was changed. The material obtained from the pulp of one tooth was subjected to further culture in sterile conditions, in an incubator at 37° in an atmosphere of 5% CO2 concentration. The use of a selective medium (MSC Nutristem - Basal Medium 500 ml + Supplement Mix (Sartorius), fetal bovine serum FBS 20% Biopharm grade, gentamicin (50mg/l), fungizone) for stem cells ensures obtaining a homogeneous culture of cells isolated from the tooth pulp - dental pulp.

The all steps of the method have been described earlier in description and additionally are shown in the diagram - figure 1.

After conducting od cell culture, in order to detach cells intended for therapeutic use, a reagent for enzymatic detachment of cells without CaCl₂ and MgCl₂ was used, e.g. CTS series (Cell Therapy Systems) CTS^{™} DPBS (1X), without calcium chloride, without magnesium chloride to rinse the growth surface and CTS^{™} TrypLE^{™} Select Enzyme for enzymatic detachment of cells. The process begins with at least one rinse of the layer of cultured cells obtained in the previous stage with a PBS solution, and then incubation in reagent containing enzyme that allows the detachment of cells from the substrate until the stem cells are detached but not destroyed, e.g. for 5 minutes at 37°C, in TrypLE. Alternative reagents can also be used for the detachment process - biotechnological trypsin and PBS, if the quality requirements for obtaining cells for therapeutic use, especially GMP, are met. After the detachment time, the cells were visible under the microscope as non-adherent, i.e. they stop adhering to the substrate of the culture vessel and were not destroyed, and an equal volume of the culture medium for mesenchymal stem cells was then added to their suspension - the same medium as during the above-mentioned at the culture step - step I of the method, in order to neutralize the enzyme for detachment. The cell suspension was transferred to another centrifuge tube to obtain centrifuged cells, e.g. a conical tube and centrifuged to obtain a cell pellet, e.g. the process of centrifugation was performed at 400g for 6 minutes. Then the cells were suspended in the medium for isolation and culture of mesenchymal cells, e.g. the above-mentioned, at the first step of culture and their number was counted. Cell counting was carried out in a known manner, e.g. for counting, a sample is taken to be counted in the NucleoCounter^{®} counter.

The cells obtained in the passage process can be further cultured using a medium containing NutriSteam with the addition of 10% PBS, antibiotic and L-glutamine in bottles that have not been coated. They can also be used to produce a tissue engineering product or subjected to further research, characterization.

A minimum of 10 million cells are obtained from one isolation, which enables for the possibility of using them for therapeutic, treatment purposes. In order to characterize the isolated cells, their identification was and should be performed using the DURAClone SC Mesenchymal Stem Cell kit. The cells obtained as a result of isolation showed expression of CD73, CD90 and CD 105 antigens and lack of expression of CD34 and CD45, which was confirmed using flow cytometry and using a microscope - the coloring appropriate for the used antibody directed to CD. The images are shown in Fig. 2 and 3 - depending on the step of the method. The isolated cells can be frozen using NutriFreez cell freezing medium and the dedicated CoolBox freezing system. The frozen cells are cooled to -80°C, ensuring a freezing rate of approx. -1°C/min. Then the cryovials are placed in a vessel with liquid nitrogen, where they can be stored for a long time at -196°C.

## Claims

1. Method for obtaining stem cells from dental pulp for use in therapeutic purposes comprising the step of culture of cells obtained from dental pulp, **characterized in that** the cells are obtaining *in vitro* from extracted tooth or teeth, preferably a molar and/or a milk tooth after decontamination and obtaining the living dental pulp from tooth by cutting it at the height of cementoenamel junction in a manner to not disturbing of pulp chamber during this process, and then the pulp is incubated with addition of collagenase for enzymatic digestion, for at least 30 minutes, preferably 1 hour, with mixing, after which a known complete medium for stem cell growth is added to the obtained suspension, then centrifuged at a temperature of 4 to 20 °C, and the cell sediment is suspended in medium for cell culture of mesenchymal stem cells with addition of fetal bovine serum, gentamicin and fungizone in an amount for preventing cell culture decontamination in culture vessel, while the vessel is previously coated with the medium using a known preparation and the dental pulp cell culture is carried out at a temperature of 37°C in an atmosphere containing 5% CO2, changing the medium at most 72 hours, then after obtaining 60-80% of cell confluence during the providing of cell culture, the cells are passaged for detaching the cells using known reagents for enzymatic detachment of cells without CaCl₂ and MgCl₂ and preferably further cell culture is carried out in a culture vessel in a medium without the coating.

2. The method according to claim 1, wherein the obtained cell suspension after detachment is placed again in culture vessels with a surface area at least 3x larger than the previously used culture vessel and the cell culture is carried out until 60-80% confluence is obtained.

3. The method according to claim 1-2, wherein the pulp is incubated with the addition of collagenase enzyme for enzymatic digestion in an amount of 4 mg/ml.

4. The method according to claims 1-3, wherein the pulp is incubated with the addition of collagenase enzyme at a temperature of 37°C.

5. The method according to claims 1-4, wherein known complete medium for stem cell growth is added in an amount of 1:1 to the volume of the mixture.

6. The method according to claims 1-5, wherein known complete medium for stem cell growth is added and then centrifuged at 400g for 6 minutes.

7. The method according to claim 1-6, wherein the cell sediment is suspended in culture medium for culture of mesenchymal stem cells with the addition of fetal bovine serum in an amount of at least 20% FBS in 1 ml of the medium.
